# EUROPEAN PATENT SPECIFICATION

(11) **EP 2 058 384 B1**
(45) Date of publication and mention of the grant of the patent: **07.05.2014**
(21) Application number: 07425706.4
(22) Date of filing: 12.11.2007
(51) Int. Cl.: C12G 1/02

(54) **Fermentation method and apparatus adapted for the method**
Verfahren zur Fermentierung und Vorrichtung zur Ausführung des Verfahrens
Procédé de fermentation et appareil adapté au procédé

(43) Date of publication of application: 13.05.2009
(73) Proprietor: Noform S.r.l., 30020 Meolo (VE) (IT)
(72) Inventor: Crosato, Remo, 31048 San Biagio di Callalta (Treviso) (IT)
(74) Representative: Citron, Massimiliano

(56) References cited:
- EP-A- 1 314 778
- WO-A-98/45403
- WO-A-2004/091764
- WO-A-2005/023977
- WO-A-2006/087601

## Description

The invention regards a fermentation method and an apparatus adapted to implement the method. Though the invention is useful for the treatment of any vegetable product in the form of crushed material, the subsequent description shall refer in an exemplifying manner to winemaking, field in which the invention proved particularly efficient.

Winemaking occurs with the aid of special vats where the must is introduced for fermentation. The fermentation process generates a large amount of gaseous products, especially CO₂, which participate actively in the production of good quality wine. The gases are released by the must and they push the marc and any other solid particles towards the top where they compact and form a solid layer, known as "cap".

Winemaking methods suitably exploit the fermentation gases. WO 2006/087601 describes a winemaking vatwhich controls the pressure of the gases within it, regulating it to an almost constant value.

In WO 98/45403 the gases are exploited to remix the cap and prevent it from solidifying. A fermenter vat is described, provided within it with an inclined diaphragm under which the gas accumulates and then constantly flows, in the form of bubbles, towards the floor of the cap. The bubbles agitate and keep it mixed. A valve and an external pipe allow quick evacuation of the gas accumulated under the diaphragm to discharge it under the cap.

This invention has various disadvantages including the fact that:
- the gas volume accumulated depends on the size of the diaphragm, and it depends on the overall volume of the fermenter;
- as the gas accumulates, the cap raises upwards, and it requires supervision to avoid overflowing;
- even the marc finds its way under the diaphragm, thus there it can encrust creating serious cleaning problems, it can clog the pipe and the external valve; and definitely limits the amount of gas which can be accumulated.

EP 1 314 778 describes a system of two vats connected by pipes equipped with valves. Must is discharged from the 1st to the 2nd vat to let it produce fermentation gas, whose pressure is then explited to spill must over the marc cap.

The object of the present invention is to provide a method and an apparatus for the treatment of a vegetable product in the form of crushed material, preferably must, capable of exploiting gases produced during fermentation without the above-mentioned drawbacks.

Such object is obtained by means of a method as claimed in Claim 1.

Thus, exploiting the natural gas generated by the fermentation, the cap can be broken.

The preferred variants, separate or combined, of the method are listed in the dependent method Claims.

The method of the invention can be implemented by an apparatus as claimed in Claim 6.

Preferred variants, separate or combined, of the apparatus are listed in the dependent apparatus Claims.

The apparatus can comprise a programmable processing device programmed for controlling the valve means as defined in Claims 10 to 14.

The invention also regards a program for the said programmable device such that, when loaded into and run by the programmable device, the program man-ages the control of said valve means and/or reading of the said sensors.

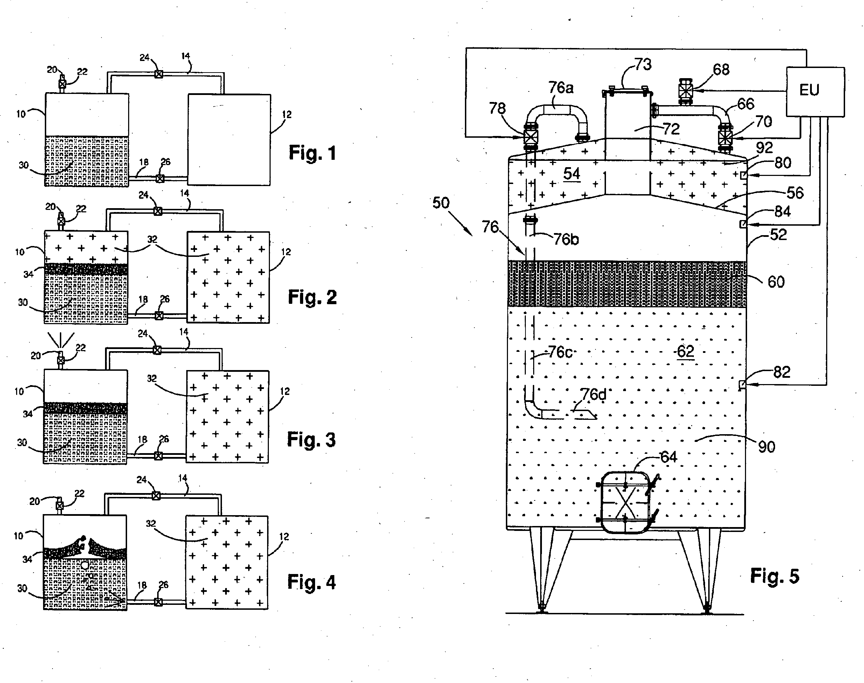

Now, reference shall be made to figures 1-4, which show the principle of the invention by means of a diagram.

A vat or winemaking apparatus 10 (fig. 1) is filled with must 30 through known means and methods. A pipe 14 puts into communication in a controllable manner, by means of a valve 24, the upper part of the vat 10 to the upper part of a second vat 12. A second pipe 18 puts into communication in a controllable manner, by means of a valve 26, the lower part of the vat 10 to the upper part of the vat 12. The vat 10 has an upper vent 20 controllable by means of a valve 22. The valve 26 is initially closed in such a manner to prevent backflow of the must 30.

After a given period of time (fig. 2) the must 30 under fermentation generates gaseous products or gas 32 (especially CO₂) and leads to the formation of a solid cap 34. It should be observed that the pipe 18 ends up into the vat 10 at a point occupied by the must 30, at a level below the bottom of the cap 34. The valve 24 is opened and the gas 32 spontaneously flows into the vat 12. The valve 22 is closed to prevent gas 32 from leaking from the vat 10.

After a preset period of time, the vats 10, 12 are isolated by closing the valve 24. The gas 32 remains trapped under pressure in the vat 12, while the other is depressurised (or degassed) through the pipe 20 by opening the valve 22 (fig. 3).

The pressure of the gas 32 in the isolated vat 10 can be controlled in the preceding step by means of the valve 22, for example using one having an opening calibrated threshold. A value proved experimentally advantageous ranges between 0.3 and 2 bars (such value shall be established from time to time by the user and depending on the type of grapes/must alongside the final product the winemaker strives to obtain: therefore, should the products treated according to this invention require it, higher or lower pressures with respect to the ones indicated and usually used could be applied). The valve 22 also allows the adjustment of the residue pressure in the vat 10 after degassing; re-establishing the atmospheric pressure was proved experimentally advantageous and simple.

Lastly, (fig. 4), the valve 26 is opened and the gas 32 spontaneously flows into the must 30. Rising, the gas 32 shall determine delicate breakage of the cap 34 and its leaching extracting its aromas and natural pigments.

Advantageously the components of figures 1-4 are integrated in a single winemaking apparatus, described below with reference to the drawings attached, wherein
Figures 1-4 show the steps of the method according to the invention;
Fig. 5 shows a preferred embodiment of the winemaking apparatus, indicated with 50;
Fig. 6 shows a second preferred embodiment of the winemaking apparatus, indicated with 150.

The winemaking apparatus 50 for holding must 90 is made up of a cylindrical shell 52 partitioned internally by a separation wall 56 shaped as a horizontal dome, into two sub-vats or superimposed volumes 54, 62, respectively upper and lower.

The lower volume 62 communicates to the outside by means of a base hatch 64 and a vertical hatchway 72, around which the vat 54 develops.

The upper vat 54 can be connected selectively to the hatchway 72 by means of a pipe 66 and a valve 70. The same pipe 66 is provided with a second valve 68 with the function of putting into communication the hatchway 72 to the outside (when its closing door 73 is closed).

The upper vat 54 can also be connected selectively to the lower vat 62 through a pipe 76 and a valve 78. The pipe 76 has an elbow section 76a which runs external to the shell 52, a vertical section 76b which runs through the upper vat 54, a vertical section 76c which passes through part of the lower vat 62 and a horizontal outlet section 76d.

The length of section 76c is such that, during the steps of the method, section 76d is always, also taking into account the expected level of the must 90, under the cap generated by the fermentation, indicated with 60. However, an adapting adjustment system, for example a telescopic pipe 76d which can be controlled from outside to adjust the height or level of section 76d, can be installed.

Control of the telescopic pipe or equivalent means for conveying the gas provided with a gas outlet with adjustable position in the in vat 62 can occur by means of a programmable processing device EU, for example a PC or PLC. This device can be provided with timers, programming user interfaces and driving stages for the valves 68, 70, 78 (see arrows in fig. 5).

The device EU can be interfaced with a pressure sensor 80, which measures the pressure of the gas 92 in vat 54, and/or a pressure sensor 84, which measures the pressure of the gas present in vat 62, and/or a level sensor 82, which measures the level of the liquid in the vat 62.

The winemaking apparatus 50 operates as follows. Reference shall also be made to figures 1-4 for correspondence with the details provided beforehand. In particular observe the correspondence between:
Vat 10 ↔ Vat 62
Vat 12 ↔ Vat 54
Valve 22 ↔ Valve 68
Valve 24 ↔ Valve 70
Valve 26 ↔ Valve 78.
   (i) Vat 62 is filled with must 90.
   (ii) After a given period of time the must 90 under fermentation generates gas and a solid cap 60. Valve 70 is opened, valve 68 shall be regulated in such a manner to bring the two vats to a preset pressure and maintain it constant over a given period of time, valve 78 is closed. Thus, spontaneously, the gas flows into the vat 54 (see reference 92 in fig. 5).
   (iii) After a set period of time, valve 70 is closed. The gas 92 remains trapped under pressure in the vat 54, while the other vat 62 is depressurised (or degassed) by opening the valve 68.
   (iv) The valve 78 opens and the gas 92 spontaneously flows into the must 30 and while rising it interacts with the cap 60, breaking it.

The steps of the process described above are preferably controlled by the device EU.

By reading and processing the data delivered by the sensor 80, the device EU can control the valves 68, 70, 78 to perform the steps of the method when for example a maximum threshold pressure is reached in the vat 54.

By reading and processing the data delivered by the sensor 82, the device EU can control the said telescopic pipe or equivalent means to convey in such a manner that in step (iv) it is always ensured that the gas 92 runs into the cap 60 from beneath. However, it can be attained that the cap 60 is hit by the gas 92 even for example at a point within its thickness.

It is clear that each step of the winemaking process of the invention can be advantageously automated and/or programmed. This allows, for example, to set periodic cycles of breakage of the cap 60, to experiment various methods of degassing the vat 10 (finding the best intermediate pressure value between the one in the vat 54 and the room pressure: the required value is obtained by controlling the data of the sensor 84 through the device EU), to program the parameters for releasing the gas 92 into the vat 10 (such as the duration of the single-discharge release or the duration of various consecutive impulses, the gas flow-rate per each impulse, etc.).

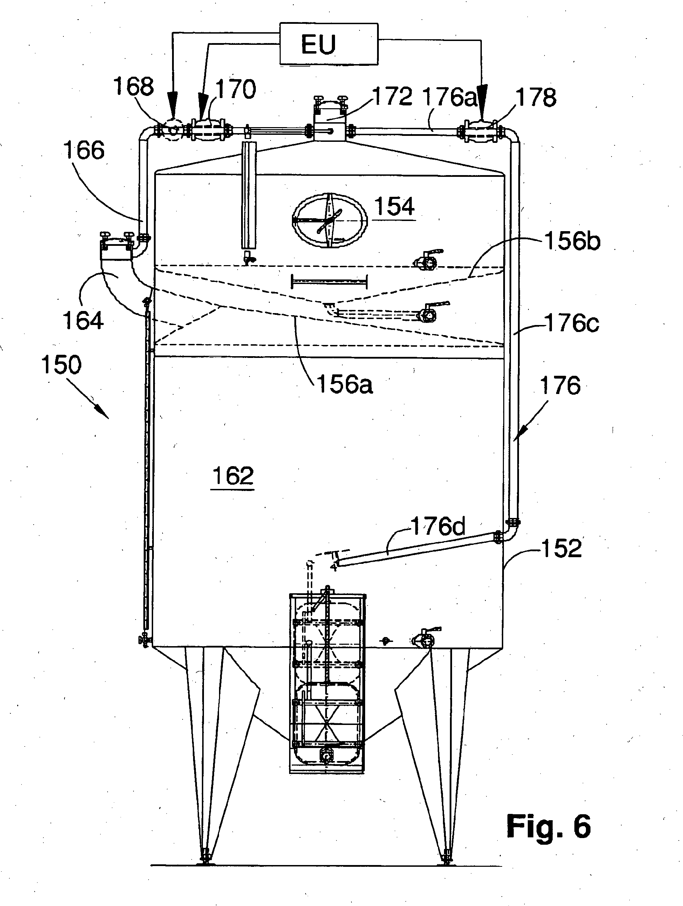

A second winemaking apparatus 150 is shown in fig. 6. Its operation is identical to the previous one, thus only components substantially deferring from it in terms of construction (for example must and cap are not shown) will be indicated. Elements operatively analogous to the previous ones are indicated by the suffix "1". In practice, the piping systems conveying gas into and out of the gas gathering vat and the internal repartition of the external shell are made in a different manner.

The winemaking apparatus 150 is made up of a cylindrical shell 152 repartitioned internally by two separation walls having a horizontal dome 156a, b into two sub-vats or superimposed volumes 154, 162, respectively upper and lower. The lower volume 162 communicates with the outside through a vent 164.

The upper vat 154 is selectively connectable to the vent 164 through a pipe 166 and a valve 170.

A valve 168 on the pipe 166 has the function of making communicating the vent 164, and thus the vat 162, to the outside and of regulating the pressures inside the vats.

The upper vat 154 is also selectively connectable to the lower vat 162 through a pipe 176 and a valve 178. The pipe 176 has an elbow section 176a which runs above and external to vat 154, a vertical section 176b which runs external to vats 154, 162, and an almost horizontal outlet section 176d which extends inside vat 162, with the outlet approximately at the centre of the vat itself and at a point, with the must present, beneath the cap.

As described previously, a programmable device EU manages the automatic driving of the valves 168, 170, 178.

For simplicity purposes, the pressure and/or level sensors described above, which perform the same functions as already mentioned beforehand, are not shown.

The operation of the winemaking apparatus 150 is conceptually analogous to the previous one and it is not repeated. Obviously, all the observations expressed regarding the winemaking apparatus 50 remain valid. In particular, the functional correspondence between the components of the winemaking apparatus 50, 150 should be observed:
Vat 62 ↔ Vat 162 (must containment)
Vat 54 ↔ Vat 154 (gas containment)
Valve 68 ↔ Valve 168 (for degassing the must containing vat)
Valve 70 ↔ Valve 170 (for gathering gas between the two vats)
Valve 78 ↔ Valve 178 (for discharging gas form one vat to the other).

## Claims

1. Method for the treatment of a vegetable product in the form of crushed material comprising the steps of:
(i) Storing the crushed material in a first vat (10) to let it ferment and form a cap (34) of solid particles floating on a liquid mass (30) therein;
(ii) Connecting a second vat (12) to the first for gathering the gaseous products (32) of fermentation in it;
(iii)Isolating the two vats;
(iv) Reducing the gaseous pressure in the first vat;
(v) Connecting the second vat to the first at a point beneath the cap so that, due to the differential pressure between the two vats, spontaneous pouring off of the gaseous products in the liquid mass occurs, in such a manner that while rising they get into contact with the cap.

2. Method according to claim 1, wherein steps (ii) to (v) are performed cyclically according to a preset program.

3. Method according to any one of the preceding claims, wherein in step (iv) the gaseous pressure in the first vat is reduced until it reaches room pressure or the gaseous pressure in the first vat is reduced until it reaches an intermediate pressure value between the one of the second vat and the room pressure.

4. Method according to any one of the preceding claims, wherein in step (v) pouring off is performed in only one time, substantially involving all the content of the second vat or through repeated preset-flow impulses, involving from time to time fractions of the content of the second vat.

5. Method according to any one of the preceding claims, wherein the gaseous pressure in the second vat is controlled and step (v) is performed when such pressure exceeds a preset threshold.

6. Apparatus (50) for the treatment of a vegetable product in the form of crushed material suitable for the implementation of the method of the preceding claims, comprising
- A first vat (62) for containing the crushed material (90) and a second collection vat (54) for gathering gaseous products (92) generated in the first vat from the crushed material's fermentation,
- A first piping system (66) adapted to let communicate a part of the first vat, where gaseous products are gathered, with the second vat, (#)
- A second piping system (76) adapted to let communicate the second and the first vat, the system being provided with an outlet in the first 40 vat where, in use, the liquid mass of the crushed material is present,
- First and second valve means (70, 78) respectively associated to the first and second piping system to make the two vats selectively communicating depending on the open/dosed status of said means.

7. Apparatus according to claim 6, comprising third valve means (68) for degassing the first vat towards the outside.

8. Apparatus according to claim 6 or 7, comprising an external shell (52) partitioned internally by at least one separation wall (56) into two sub-volumes which constitute said first and second vat and
wherein said first and second vat are arranged vertically one over the other inside the shell.

9. Apparatus according to claim 8, comprising two independent separation walls for delimiting said two sub-volumes, the two walls being concave and arranged with the concavities facing opposite directions.

10. Apparatus according to any one of the preceding claims 6 to 9, comprising a programmable processing device (EU) programmed to control the valve means (68, 70, 78) in such a manner to perform, after the loading of a vegetable product in form of crushed material into the first vat, the steps of
- Connecting the second vat to the first for gathering into it the gaseous products generated by fermentation by opening the first valve means;
- Closing the first valve means to isolate the two vats;
- Opening the second valve means to connect the second vat to the first one in such a manner that spontaneous pouring off of the gaseous products in the liquid mass of the crushed material occurs.

11. Apparatus according to claim 10, wherein the processing device (EU) is programmed to reduce the gaseous pressure in the first vat by operating the third valve means.

12. Apparatus according to one of claims 10 to 11, wherein the processing device (EU) is programmed to open the second valve means only one time, in such a manner that the gases migrate from a vat to the other substantially involving the entire content of the second vat or
wherein the processing device (EU) is programmed to open the second valve means at repeated impulses in such a manner that the gases migrate from one vat to the other through preset flow-rate packets.

13. Apparatus according to one of claims 10 to 12, wherein the processing device is interfaced with a pressure sensor (80), which measures the pressure of the gas (92) in the first vat (54), and/or a pressure sensor (84), which measures the pressure of the gas present in the second vat (62), and/or a level sensor (82), which measures the level of liquid in the second vat (62).

14. Apparatus according to claim 13, wherein the processing device (EU) is programmed to control the gaseous pressure in the second vat through the related sensor and control the opening of the second valve means when such pressure exceeds a preset threshold.

15. Program for a programmable device as in claims 10 to 14 such that, when it is loaded and run in the programmable device, it manages the control of the valve means and/or the reading of the sensors.

## Patentansprüche

1. Methode für die Behandlung eines pflanzlichen Produkts in Form von zerkleinertem Material, welche die folgenden Schritte umfasst:
(i) Aufbewahrung des zerkleinerten Materials in einem ersten Bottich (10) für die Fermentation und zur Bildung eines Huts (34) aus festen Partikeln, die auf der hier enthaltenen flüssigen Masse (30) schwimmen;
(ii) Anschluss eines zweiten Bottichs (12) an den ersten zum Sammeln des dort entstehenden gasförmigen Fermentationsprodukts (32);
(iii) Isolierung der beiden Bottiche;
(iv) Verringerung des Gasdrucks im ersten Bottich;
(v) Anschluss des zweiten Bottichs an den ersten Bottich an einer Stelle unter dem Hut, so dass, aufgrund des Druckdifferentials zwischen den beiden Bottichen, es zu einem spontanen Ausfließen der gasförmigen Produkte in der flüssigen Masse kommt, so dass diese bei Anstieg in Berührung mit dem Hut kommen.

2. Methode gemäß Anspruch 1, wo die Schritte (ii) bis (v) zyklisch nach einem voreingestellten Programm ausgeführt werden.

3. Methode gemäß einem beliebigen der vorhergehenden Ansprüche, wo bei Schritt (iv) der Gasdruck im ersten Bottich reduziert wird, bis er den Umgebungsdruck erreicht oder der Gasdruck im ersten Bottich reduziert wird, bis er einen Druckwert erreicht, der zwischen dem des zweiten Behälters und dem Umgebungsdruck liegt.

4. Methode gemäß einem beliebigen der vorhergehenden Ansprüche, wo der Schritt (v) zum Ablassen nur einmal stattfindet und dabei im Wesentlichen den ganzen Inhalt des zweiten Bottichs involviert oder über voreingestellte Fließimpulse, wo von Zeit zu Zeit Fraktionen des zweiten Bottichs betroffen sind.

5. Methode gemäß einem beliebigen der vorhergehenden Ansprüche, wo der Gasdruck im zweiten Bottich kontrolliert und Schritt (v) ausgeführt wird, wenn dieser Druck über einen bestimmten Schwellenwert ansteigt.

6. Apparat (50) zur Behandlung eines pflanzlichen Produkts in Form zerkleinerten Materials für die Implementierung der Methode der zuvor genannten Ansprüche, wozu gehören
- ein erster Bottich (62) für das zerkleinerte Material (90) und ein zweiter Sammelbottich (54) zum Sammeln der gasförmigen Produkte (92), die im ersten Bottich durch die Fermentation des zerkleinerten Materials entstehen,
- ein erstes Rohrleitungssystem (66), das so angepasst ist dass ein Teil des ersten Bottichs, wo das gasförmige Produkt gesammelt wird, mit dem zweiten Bottich kommuniziert, so dass das gasförmige Produkt unter Druck im zweiten Bottich verweilt,
- ein zweites Rohrleitungssystem (76), das so angepasst ist, dass der zweite und der erste Bottich miteinander kommunizieren, wobei das System mit einem Auslass im ersten Bottich versehen ist, wo sich - bei Verwendung - die flüssige Masse des zerkleinerten Materials befindet,
- eine erste und zweite Ventilanlage (70, 78), die jeweils mit dem ersten und dem zweiten Rohrleitungssystem verknüpft sind, damit die beiden Bottich selektiv je nach dem Zustand der besagten Anlagen kommunizieren können.

7. Apparat gemäß Anspruch 6, mit einer dritten Ventilanlage (68) zum Entgasen des ersten Bottichs an die Umgebung.

8. Apparat gemäß Anspruch 6 oder 7, mit einer externen Hülle (52), die innen durch mindestens eine Trennwand (56) in zwei Teilvolumen unterteilt ist, welche den ersten und den zweiten Bottich darstellen und wo der besagte erste und zweite Bottich innerhalb der Ummantelung vertikal übereinander angeordnet sind.

9. Apparat gemäß Anspruch 8, mit zwei unabhängigen Trennwänden zur Begrenzung der besagten Teilvolumen, wobei diese beiden Wände konkav sind und deren Einbuchtungen nach den gegenüberliegenden Seiten ausgerichtet sind.

10. Apparat gemäß einem der vorhergehenden Ansprüche 6 bis 9, mit einem programmierbaren Verarbeitungsgerät (EU), das zur Steuerung der Ventilanlagen (68, 70, 78) programmiert ist, dergestalt, dass es nach dem Füllen des pflanzlichen Produkts in zerkleinerter Form in den ersten Bottich die folgenden Schritte ausführt:
- Verbindung des zweiten mit dem ersten Bottich, um dort bei Öffnen der ersten Ventilanlage die gasförmigen Produkte zu sammeln, die durch die Fermentation entstanden sind;
- Das Schließen der ersten Ventilanlage bedeutet das Trennen der beiden Bottiche;
- Das Öffnen der zweiten Ventilanlage bedeutet, dass der zweite Bottich mit dem ersten Botich verbunden wird, so dass ein spontanes Abfließen der gasförmigen Produkte in der flüssigen Masse des zerkleinerten Materials erfolgt.

11. Apparat gemäß Anspruch 10, wo das Prozesssteuergerät (EU) so programmiert ist, dass der Gasdruck in dem ersten Bottich durch Betätigen der dritten Ventilanlage reduziert wird.

12. Apparat gemäß einem der Ansprüche 10 bis 1l, wo das Prozesssteuergerät (EU) so programmiert ist, dass die zweite Ventilanlage nur ein Mal geöffnet wird, so dass die Gase von einem Bottich in den anderen strömen und dabei den gesamten Inhalt des zweiten Bottichs betreffen oder wo das Prozesssteuergerät (EU) so programmiert ist, dass die zweite Ventilanlage mit wiederholten Impulsen geöffnet wird, so dass die Gase über voreingestellten Durchflussraten von einen in den anderen Bottich strömen.

13. Apparat gemäß einem der Ansprüche 10 bis 12, wo das Prozesssteuergerät eine Schnittstelle auf einen Drucksensor (80) hat, der den Gasdruck (92) im ersten Bottich (54) misst und/oder einen Drucksensor (84), der den Gasdruck im zweiten Bottich (62) misst und/oder einen Pegelsensor (82), der den Flüssigkeitspegel im zweiten Bottich (62) misst.

14. Apparat gemäß Anspruch 13, wo das Prozesssteuergerät (EU) so programmiert ist, dass es den Gasdruck im zweiten Bottich durch den verbundenen Sensor und ie Steuerung der Öffnung der zweiten Ventilanlage steuert, wenn dieser Druck eine voreingestellte Schwelle übersteigt.

15. Programm für ein programmierbares Gerät nach den Ansprüchen 10 bis 14, damit dieses nach dem Laden und Laufen auf dem programmierbaren Gerät die Ventilanlagen und/oder das Ablesen der Sensoren steuert.

## Revendications

1. Méthode de traitement d'un produit végétal sous une forme écrasée, comprenant les étapes de :
(i) Stockage du produit écrasé dans une première cuve (10) pour le laisser fermenter et former une couche (34) de particules solides flottant sur une masse liquide (30) ;
(ii) Raccordement d'une deuxième cuve (12) à la première pour collecter les substances gazeuses (32) issues de la fermentation interne ;
(iii) Isolation de deux cuves ;
(iv) Réduction de la pression gazeuse dans la première cuve ;
(v) Raccordement de la deuxième cuve à la première en un point sous la couche, de sorte qu'il se produise un écoulement spontané des substances gazeuses dans la masse liquide suite à la pression différentielle entre les deux cuves, et de sorte que ces substances touchent la couche pendant la montée.

2. Méthode selon la revendication 1, dans laquelle les étapes (ii) à (v) sont accomplies cycliquement selon un programme préétabli.

3. Méthode selon n'importe laquelle des revendications précédentes, dans laquelle la pression gazeuse dans la première cuve à l'étape (iv) est réduite jusqu'à atteindre la pression ambiante, ou la pression gazeuse dans la première cuve est réduite jusqu'à atteindre une valeur de pression intermédiaire entre celle de la deuxième cuve et la pression ambiante.

4. Méthode selon n'importe laquelle des revendications précédentes, dans laquelle la coulée à l'étape (v) a lieu en une seule fois, en touchant fondamentalement tout le contenu de la deuxième cuve, ou en touchant de temps à autre des portions du contenu de la deuxième cuve par impulsions réitérées à débit préétabli.

5. Méthode selon n'importe laquelle des revendications précédentes, dans laquelle la pression gazeuse dans la deuxième cuve est contrôlée et l'étape (v) se produit lorsqu'une telle pression dépasse un seuil préétabli.

6. Appareil (50) pour le traitement d'un produit végétal sous la forme de produit écrasé adapté à la mise en oeuvre de la méthode des revendications précédentes, comprenant
- une première cuve (62) pour contenir le produit écrasé (90) et une deuxième cuve (54) pour collecter les substances gazeuses (92), qui sont issues de la fermentation du produit écrasé dans la première cuve,
- un premier système de tuyauterie (66) apte à permettre la communication d'une partie de la première cuve, où sont collectées les substances gazeuses, avec la deuxième cuve, de sorte que les substances gazeuses peuvent rester emprisonnées sous pression dans la deuxième cuve,
- un deuxième système de tuyauterie (76) apte à permettre la communication entre la deuxième cuve et la première cuve, le système étant muni d'une sortie dans la première cuve, où se trouve la masse liquide de produit écrasé pendant l'emploi,
- les premier et deuxième dispositifs de vannes (70, 78) associés respectivement au système de première et deuxième tuyauteries pour mettre en communication les deux cuves en fonction de l'état d'ouverture/fermeture dudit dispositif.

7. Appareil selon la revendication 6, comprenant un troisième dispositif de vanne (68) pour le dégazage de la première cuve vers l'extérieur.

8. Appareil selon la revendication 6 ou 7 comprenant un logement extérieur (52) divisé intérieurement par une cloison de séparation (56) au minimum, en deux sous-volumes, lesquels forment lesdites première et deuxième cuves, et où lesdites première et deuxième cuves sont aménagées verticalement l'une au-dessus de l'autre dans le logement.

9. Appareil selon la revendication 8 comprenant deux cloisons indépendantes pour la délimitation desdits deux sous-volumes, les deux cloisons étant concaves et aménagées avec les concavités orientées dans deux directions opposées.

10. Appareil selon n'importe laquelle des revendications précédentes de 6 à 9 comprenant un dispositif de traitement programmable (UE) programmé pour contrôler le dispositif de vanne (68, 70, 78) de façon à accomplir les étapes suivantes, après le remplissage d'un produit végétal sous forme écrasée dans la première cuve,
- Raccordement de la deuxième cuve à la première pour collecter les substances gazeuses issues de la fermentation par l'ouverture du premier dispositif de vanne ;
- fermeture du premier dispositif de vanne pour isoler les deux cuves ;
- ouverture du deuxième dispositif de vanne pour raccorder la deuxième cuve à la première de telle manière que se produise l'écoulement spontané des substances gazeuses dans la masse liquide de produit écrasé.

11. Appareil selon la revendication 10, dans lequel le dispositif de traitement (UE) est programmé pour réduire la pression gazeuse dans la première cuve par l'actionnement du troisième dispositif de vanne.

12. Appareil selon n'importe laquelle des revendications 10 à 11, dans lequel le dispositif de traitement (UE) est programmé pour ouvrir une seule fois le deuxième dispositif de vanne, de sorte que les gaz passent d'une cuve à l'autre en touchant fondamentalement le contenu entier de la deuxième cuve, ou dans lequel le dispositif de traitement (UE) est programmé pour ouvrir le deuxième dispositif de vanne par impulsions réitérées, de sorte que les gaz passent d'une cuve à l'autre par quantités de débit préétablis.

13. Appareil selon n'importe laquelle des revendications 10 à 12, dans lequel le dispositif de traitement est raccordé à un capteur de pression (80), qui mesure la pression du gaz (92) dans la première cuve (54), et/ou un capteur de pression (84), qui mesure la pression du gaz se trouvant dans la deuxième cuve (62), et/ou un capteur de niveau (82), qui mesure le niveau du liquide dans la deuxième cuve (62).

14. Appareil selon la revendication 13, dans lequel le dispositif de traitement (UE) est programmé pour contrôler la pression gazeuse dans la deuxième cuve par le biais du capteur respectif et contrôler l'ouverture du deuxième dispositif de vanne lorsqu'une telle pression dépasse un seuil préétabli.

15. Programme destiné à un dispositif programmable selon les revendications 10 à 14, qui gère le contrôle du dispositif de vanne et/ou la lecture des capteurs lorsqu'il est chargé et fonctionne dans ledit dispositif programmable.
